(19) Europäisches Patentamt
European Patent Office
Office européen des brevets

(11) **EP 4 773 144 A1**

(12) **EUROPEAN PATENT APPLICATION**
published in accordance with Art. 153(4) EPC

(43) Date of publication:
**08.07.2026 Bulletin 2026/28**

(51) International Patent Classification (IPC):
***G16B 40/10*** (2019.01)

(21) Application number: 23951340.1

(22) Date of filing: **29.11.2023**

(86) International application number:
**PCT/CN2023/135133**

(87) International publication number:
**WO 2025/050527 (13.03.2025 Gazette 2025/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **06.09.2023 CN 202311146735**

(71) Applicants:
• **Qitan Technology Ltd., Beijing
Beijing 100192 (CN)**
• **Qitan Technology Ltd., Chengdu
Chengdu, Sichuan 610041 (CN)**

(72) Inventors:
• **HAO, Junlong
Beijing 100192 (CN)**
• **WEI, Qiang
Beijing 100192 (CN)**
• **ZHUO, Yuan
Beijing 100192 (CN)**
• **TIAN, Limin
Beijing 100192 (CN)**
• **LI, Rui
Beijing 100192 (CN)**

(74) Representative: **Laqua, Bernd Christian Kurt
Wuesthoff & Wuesthoff
Patentanwälte und Rechtsanwalt PartG mbB
Schweigerstraße 2
81541 München (DE)**

(54) **SEQUENCE PROCESSING METHOD AND APPARATUS, POSTERIOR MATRIX PROCESSING METHOD AND APPARATUS, AND COMPUTING DEVICE**

(57) The present application relates to the field of nanopore sequencing, and in particular, to a method and apparatus for processing a first vectorized representation sequence representing a polymer, a method and apparatus for processing a posterior matrix, a computing device, a computer-readable storage medium, and a computer program product. The method for processing a first vectorized representation sequence representing a polymer comprises: inputting a first vectorized representation sequence into a first machine learning model to obtain a weight vector output by the first machine learning model, wherein the weight vector has the same length as the first vectorized representation sequence and comprises a plurality of predicted translocation values, and each predicted translocation value indicates how many polymer units a polymer has moved at a corresponding time step relative to an observation site of nanopore sequencing; and integrating the first vectorized representation sequence and the weight vector to obtain a second vectorized representation sequence representing the polymer.

FIG. 3

Processed by Luminess, 75001 PARIS (FR)

## Description

## TECHNICAL FIELD

The present application relates to the field of nanopore sequencing, and in particular, to a method and an apparatus for processing a first vectorized representation sequence characterizing a polymer, a processing method and an apparatus for processing a posterior matrix, a computing device, a computer-readable storage medium, and a computer program product.

## BACKGROUND

In the prior art, nanopore signals can be processed by machine learning methods to output corresponding polymer sequences.

The methods described in this section are not necessarily methods that have been previously conceived or employed. Unless otherwise indicated, any method described in this section should not be assumed to be prior art only because it is included herein. Similarly, unless otherwise indicated, the problems referred to in this section should not be considered to have been recognized in any prior art.

## SUMMARY

The present disclosure provides a method and an apparatus for processing a first vectorized representation sequence characterizing a polymer, a processing method and an apparatus for processing a posterior matrix, a computing device, a computer-readable medium, and a computer program product.

According to an aspect of the present disclosure, a method for processing a first vectorized representation sequence characterizing a polymer is provided, where the polymer includes a polymer unit sequence, the first vectorized representation sequence is generated based on a raw signal derived from the polymer by performing nanopore sequencing on the polymer, and the method includes: inputting the first vectorized representation sequence into a first machine learning model to obtain a weight vector output by the first machine learning model, where the weight vector has the same length as the first vectorized representation sequence and includes a plurality of predicted translocation values, and each predicted translocation value indicates how many polymer units the polymer has moved relative to an observation site of the nanopore sequencing at a corresponding time step; and integrating the first vectorized representation sequence with the weight vector to obtain a second vectorized representation sequence characterizing the polymer. According to another aspect of the present disclosure, a processing method for processing a posterior matrix is provided, where the posterior matrix is obtained by processing, by a third machine learning model, the second vectorized representation sequence obtained according to the method for processing a first vectorized representation sequence characterizing a polymer provided by the present disclosure, the number of rows of the posterior matrix is equal to the number of polymer unit classifications, elements in each column of the posterior matrix indicate one corresponding polymer unit among a plurality of polymer units, elements in a plurality of consecutive columns of the posterior matrix indicate corresponding different polymer units in the plurality of polymer units, and the processing method includes: decoding the posterior matrix to obtain a decoded sequence, each element in the decoded sequence corresponding to one polymer unit classification of the polymer unit classifications; and obtaining the polymer unit sequence based on the decoded sequence.

According to yet another aspect of the present disclosure, a computing device is provided, including: at least one processor; and at least one memory having one or more code instructions stored thereon, where the one or more code instructions, when executed by the at least one processor, cause the computing device to perform the method for processing a first vectorized representation sequence characterizing a polymer or the processing method for processing a posterior matrix provided by the present disclosure.

According to still another aspect of the present disclosure, a non-transitory computer-readable storage medium having one or more computer instructions stored thereon is provided, where the one or more computer instructions are used to cause a computer to perform the method for processing a first vectorized representation sequence characterizing a polymer or the processing method for processing a posterior matrix provided by the present disclosure.

According to yet still another aspect of the present disclosure, a computer program product is provided, including one or more computer programs, wherein the one or more computer programs, when executed by a processor, cause the processor to implement the method for processing a first vectorized representation sequence characterizing a polymer or the processing method for processing a posterior matrix provided by the present disclosure.

According to embodiments of the present disclosure, the output of the base translocation prediction module can be integrated with high-dimensional information obtained by encoding the raw signal of nanopore sequencing to obtain high-dimensional features relating to the raw signal, thereby facilitating a downstream module in outputting a polymer sequence associated with the nanopore signal. By virtue of the utilization of the sequence context information during the processing procedure, improved efficiency and accuracy in processing the nanopore signal are achieved. In addition, after decoding the posterior matrix, each time step is assigned a classification representing one base or base fragment, and consecutive time steps do not represent the same base or base fragment, thereby effectively avoiding possible deletion of polymer units during decoding of the processed signal characterizing the polymer, and improving decoding efficiency and accuracy.

It should be understood that the content described in this section is not intended to identify key or important features of the embodiments of the present disclosure, nor is it intended to limit the scope of the present disclosure. Other features of the present disclosure will become readily apparent from the following description.

## BRIEF DESCRIPTION OF THE DRAWINGS

The accompanying drawings exemplarily illustrate the embodiments and constitute a part of the specification, and they are used together with the textual description of the specification to explain exemplary implementations of the embodiments. The illustrated embodiments are provided for illustrative purposes only and do not limit the scope of the claims. In all the accompanying drawings, the same reference numerals refer to similar, but not necessarily identical, elements.

FIG. 1 illustrates a schematic diagram of an exemplary system in which various methods described herein may be implemented according to an embodiment of the present disclosure;

FIG. 2 illustrates a flowchart of a method for processing a first vectorized representation sequence characterizing a polymer according to an exemplary embodiment of the present disclosure;

FIGS. 3 and 4 illustrate block diagrams of exemplary processes for processing a first vectorized representation sequence characterizing a polymer according to an exemplary embodiment of the present disclosure;

FIG. 5 illustrates a flowchart of a processing method for processing a posterior matrix according to an exemplary embodiment of the present disclosure;

FIGS. 6 and 7 illustrate block diagrams of exemplary processes for processing a posterior matrix according to an exemplary embodiment of the present disclosure;

FIGS. 8 to 11 illustrate diagrams of exemplary processes for decoding a posterior matrix according to an exemplary embodiment of the present disclosure;

FIG. 12 illustrates a block diagram of an apparatus for processing a first vectorized representation sequence characterizing a polymer according to an exemplary embodiment of the present disclosure;

FIG. 13 illustrates a block diagram of a processing apparatus for processing a posterior matrix according to an exemplary embodiment of the present disclosure; and

FIG. 14 illustrates a structural block diagram of an exemplary electronic device capable of implementing embodiments of the present disclosure.

## DETAILED DESCRIPTION

Exemplary embodiments of the present disclosure are described below with reference to the accompanying drawings, in which various details of embodiments of the present disclosure are included to assist understanding, and these embodiments are to be considered as only exemplary.

Accordingly, it will be appreciated by those of ordinary skills in the art that various changes and modifications may be made to the embodiments described herein without departing from the scope of the present disclosure. Similarly, for clarity and conciseness, the descriptions below omit explanations of well-known functions and structures.

In the present disclosure, unless otherwise specified, the terms "first", "second", and the like, are used to describe various elements and are not intended to define a location relationship, a temporal relationship, or an importance relationship of these elements, and such terms are used only to distinguish one element from another. In some examples, a first element and a second element may refer to the same instance of the element, while in some cases they may refer to different instances based on the context of the description.

The terms used in the description of the various examples described in the present disclosure are for the purpose of describing particular examples only and are not intended to be limiting. Unless otherwise clearly indicated in the context, if the number of elements is not specifically limited, there may be one or a plurality of elements. Further, the term "and/or" used herein encompasses any one of and all possible combinations of the listed items.

In the prior art, nanopore signals can be processed by machine learning methods to output corresponding polymer sequences. For example, techniques in the field of speech recognition may be adapted to implement nanopore signal processing. In the field of speech recognition, there are two mainstream types of models, one of which is a neural network model based on autoregressive decoding, and the other is a neural network model based on dynamic programming decoding such as connectionist temporal classification (CTC). However, the neural network model based on autoregressive decoding suffers from problems such as slow processing speed, difficulty in applying context information to forward prediction, and inapplicability of the model to long sequences. The neural network model based on dynamic programming decoding such as CTC determines during decoding whether consecutive steps are identical and emits an output only when the consecutive steps are not identical, and therefore are prone to deletion errors.

In view of this, the present disclosure provides a method for processing a first vectorized representation sequence characterizing a polymer. The method includes: first inputting the first vectorized representation sequence into a first machine learning model to obtain a weight vector output by the first machine learning model, where the weight vector has the same length as the first vectorized representation sequence and includes a plurality of predicted translocation values, and each predicted translocation value indicates how many polymer units the polymer has moved relative to an observation site of the nanopore sequencing at a corresponding time step; and then integrating the first vectorized representation sequence with the weight vector to obtain a second vectorized representation sequence characterizing the polymer. Therefore, according to the present disclosure, the output of the base translocation prediction module can be integrated with high-dimensional information obtained by encoding the raw signal of nanopore sequencing to obtain high-dimensional features relating to the raw signal, thereby facilitating a downstream module in outputting a polymer sequence associated with the nanopore signal. By virtue of the utilization of the sequence context information during the processing procedure, improved efficiency and accuracy in processing the nanopore signal are achieved.

To better understand the technical solutions provided in the embodiments of the present application, the application scenarios to which the technical solutions provided in the embodiments of the present application are applicable are briefly described below. It should be noted that the application scenarios described below are provided only for illustrating the embodiments of the present application and are not intended to limit the present application. In practical implementations, the technical solutions provided in the embodiments of the present application may be flexibly applied according to actual needs.

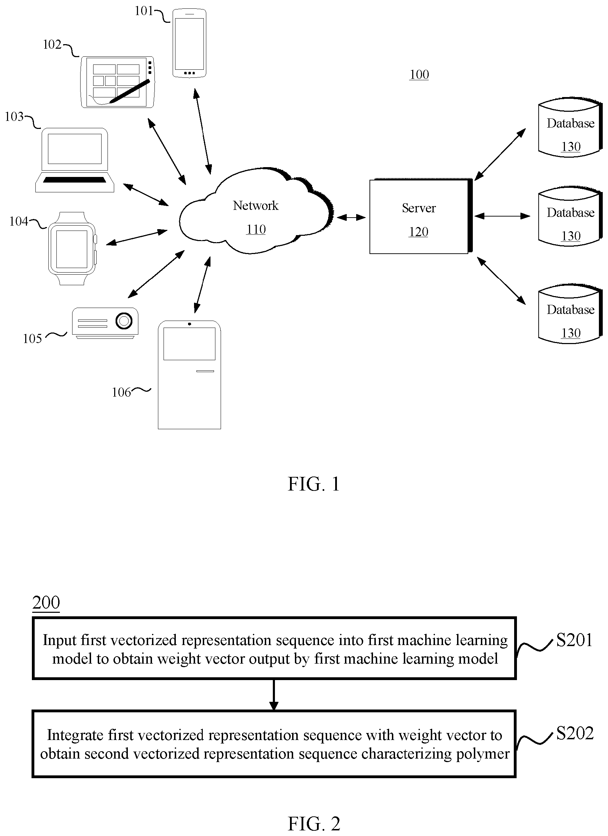

The method provided in the embodiments of the present application may be applied to the application scenario illustrated in FIG. 1. Referring to FIG. 1, an exemplary system 100 in the application scenario includes a plurality of terminal devices 101, 102, 103, 104, 105, and 106, a server 120, and one or more communication networks 110 that couple one or more client devices to the server 120. The terminal devices 101, 102, 103, 104, 105, and 106 and the server 120 may be connected and transmit data therebetween in a wired connection manner or a wireless connection manner. For example, the terminal devices 101, 102, 103, 104, 105, and 106 may be connected to the server 120 through a data cable or a wired network. The terminal devices 101, 102, 103, 104, 105, and 106 may also be connected to the server 120 through a radio frequency module, a Wi-Fi module, or a wireless network.

The terminal devices 101, 102, 103, 104, 105, and 106 may be computers, notebook computers, personal digital assistants (PDAs), tablet computers, or the like. Such terminal devices may be used by researchers, laboratory personnel, and the like in the field of nanopore sequencing applications, such that they can conveniently perform real-time monitoring, data collection, data processing, viewing and analysis of sequencing results, and other operations of polymer nanopore sequencing via the terminal devices.

The server 120 may be one server, a server cluster consisting of several servers, a cloud computing center, or a virtualization platform, or may be a personal computer, a medium- or large-scale computer, a computer cluster, or the like. The network 110 may be any type of network known to those skilled in the art that may support data communications using any one of a variety of available protocols, including but not limited to TCP/IP, SNA, IPX, and the like. By way of example only, one or more networks 110 may be a local area network (LAN), an Ethernet-based network, a token ring, a wide area network (WAN), the Internet, a virtual network, a virtual private network (VPN), an intranet, an extranet, a public switched telephone network (PSTN), an infrared network, a wireless network (e.g., bluetooth and WiFi), and/or any combination of these and/or other networks.

The system 100 may further include one or more databases 130. In some implementations, the one or more databases may be used to store data and other information. For example, one or more of the databases 130 may be used to store information such as audio files and video files. The data repository 130 may reside in various locations. For example, a data repository used by the server 120 may be located locally at the server 120, or may be remote from the server 120 and may communicate with the server 120 via a network-based or dedicated connection. The data repository 130 may be of different types. In certain embodiments, the data repository used by the server 120 may be a database, such as a relational database. One or more of these databases may store, update, and retrieve data to and from the database in response to commands.

In some implementations, one or more of the databases 130 may also be used by an application program to store application program data. The databases used by the application program may be of different types, such as a key-value store, an object store, or a conventional repository supported by a file system.

According to implementation requirements, the application scenario in the embodiments of the present application may include any number of terminal devices, servers, networks, and databases, which is not particularly limited in the present application. The method for processing a first vectorized representation sequence characterizing a polymer and the processing method for processing a posterior matrix provided in the embodiments of the present application may be performed by the server 120, or may be collaboratively performed by the terminal devices 101, 102, 103, 104, 105, and 106 and the server 120.

To further describe the technical solutions provided in the embodiments of the present application, detailed descriptions are provided below with reference to the accompanying drawings and specific implementations. Although the embodiments of the present application provide method operation steps as illustrated in the following embodiments or the accompanying drawings, more or fewer operation steps may be included in the methods based on routine practice or without creative effort. For steps in which no necessary logical causal relationship exists, the execution order of such steps is not limited to the execution order provided in the embodiments of the present application. In actual processing or when executed by an apparatus, the methods may be performed in the order shown in the embodiments or the accompanying drawings, or may be performed in parallel.

According to an aspect of the present disclosure, a method 200 for processing a first vectorized representation sequence characterizing a polymer is provided. As illustrated in FIG. 2, the method 200 includes: step S201, inputting the first vectorized representation sequence into a first machine learning model to obtain a weight vector output by the first machine learning model; and step S202, integrating the first vectorized representation sequence with the weight vector to obtain a second vectorized representation sequence characterizing the polymer.

In an example, the polymer may be any type of organic compound, such as a nucleotide or nucleic acid, a polypeptide such as a protein, or a saccharide. In an example, the polymer may be natural or synthetic.

In an example, the polymer may include a series of polymer units or a polymer unit sequence.

In the case of a nucleotide or nucleic acid, the polymer unit may be any type of nucleotide. The nucleotide may be naturally occurring or synthetic. A nucleoside is composed of a nucleobase (also referred to as a base) and a sugar. The nucleobase may include purines and pyrimidines, such as adenine, guanine, thymine, uracil, and cytosine. In an example, the nucleobase may further include a modified base. The polymer unit may also be any type of nucleic acid. The nucleic acid may generally include deoxyribonucleic acid (DNA), ribonucleic acid (RNA), cDNA, or synthetic nucleic acids known in the art, such as peptide nucleic acid (PNA), glycerol nucleic acid (GNA), threose nucleic acid (TNA), locked nucleic acid (LNA), or other synthetic polymers having nucleotide side chains.

In the case of a polypeptide, the polymer unit may be a naturally occurring or artificially synthesized amino acid.

In the case of a saccharide, the polymer unit may be a monosaccharide, a polysaccharide, or the like.

Exemplarily, the polymer may be a nucleobase-containing polymer. In this case, the polymer units may be various nucleobase identifiers representing natural nucleobase types (i.e., nucleobase IDs, e.g., adenine is represented by A, guanine is represented by G, cytosine is represented by C, thymine is represented by T, and uracil is represented by U), may be various nucleobase identifiers representing artificially synthesized nucleobases, or may be identifiers representing nucleobase fragments.

In addition, in an example, the polymer may also be any type of inorganic compound. The present disclosure does not impose any limitation in this regard.

In an example, the first vectorized representation sequence may characterize the polymer.

As used herein, the term "vectorized representation sequence" refers to a sequence composed of vectorized representations, that is, each element of the sequence is a vectorized representation, or each step of the sequence corresponds to a vectorized representation. Exemplarily, each individual vectorized representation may be a column vector having a dimension of $N \times 1$. Accordingly, a sequence composed of the individual vectorized representations (i.e., the vectorized representation sequence) has a dimension of $N \times T$, where T denotes the number of elements contained in the sequence or the number of steps of the sequence. Exemplarily, each individual vectorized representation may alternatively be a row vector having a dimension of $1 \times N$, and the resulting sequence has a dimension of $T \times N$.

In an example, the dimension N may represent the number of channels used in characterizing the information of the polymer unit. Exemplarily, N may be 126, 258, 512, or the like, and the present disclosure does not impose any limitation in this regard.

In an example, the first vectorized representation sequence may be generated based on a raw signal derived from the polymer by performing nanopore sequencing on the polymer.

As described above, the first vectorized representation sequence may characterize the polymer. Specifically, each element of the first vectorized representation sequence (i.e., each vectorized representation) is capable of characterizing a signal component (e.g., a current signal component having fluctuating characteristics) corresponding to each time step of the raw signal derived from the polymer during nanopore sequencing. That is, the effective information payload of the signal corresponding to each time step of the raw signal can be processed (e.g., encoded) into a corresponding vectorized representation of the first vectorized representation sequence.

Nanopore sequencing is a single-molecule real-time sequencing technology based on nanopore electrical signals. During the sequencing process, a DNA or RNA double strand is linked to a motor protein, and under the action of the motor protein, binds to a nanopore protein anchored on a biological membrane and is unwound. Due to the potential difference between the two sides of the biological membrane, the unwound single-stranded DNA or RNA passes through the nanopore at a certain rate. During this translocation process, the ionic current within the nanopore fluctuates as the nucleic acid moves through the pore, because different bases, due to their different chemical properties, cause different electrical signal changes when passing through the nanopore. By capturing, detecting, and analyzing the fluctuating electrical signals, subsequent base sequence identification can be enabled. That is, the nanopore-based polymer unit determination is performed based on electrical signals derived from the polymer during the sequencing process (the electrical signals having fluctuation characteristics).

In step S201 of the embodiments, the first machine learning model may be any suitable machine learning model, such as a convolutional neural network, a recurrent neural network, an attention mechanism, or a combination of one or more networks (e.g., an ensemble model). The present disclosure does not impose any limitation in this regard.

As used herein, the term "machine learning model" refers to an algorithm or mathematical function capable of transforming input data into a specific output. The model is obtained through training under a corresponding training task by receiving input training samples, providing actual output samples, and adjusting or optimizing the model based on a comparison result between the actual output samples and expected output training samples. Correspondingly, the training process or training task of the machine learning model may be summarized as a computer program updating parameter values of the model through an algorithm based on given training data and a model method so as to optimize parameters of a processing task, thereby ultimately obtaining an optimized model capable of completing recognition or prediction tasks.

In step S201, the first machine learning model may therefore refer to an algorithm or mathematical model capable of converting the first vectorized representation sequence characterizing the polymer into a weight vector. In an example, the first machine learning model may output, in response to receiving the first vectorized representation sequence, a weight vector having the same length as the first vectorized representation sequence. The weight vector may include a plurality of predicted translocation values. That is, the number of predicted translocation values included in the weight vector is the same as the number of elements or the number of steps of the first vectorized representation sequence, and is further the same as the number of steps of the raw signal derived from the polymer during nanopore sequencing.

It can be understood that when referring to the length of a vectorized representation sequence (e.g., the first vectorized representation sequence described above and the second vectorized representation sequence to be described in detail below), the term refers to the number of elements (e.g., column vectors) constituting the vectorized representation sequence. Taking the above first vectorized representation sequence having a dimension of N × T as an example, its length is T, that is, the number of elements (e.g., N × 1 column vectors) included in the first vectorized representation sequence. It can further be understood that, in the present disclosure, the terms such as "length", "number of elements", "number of steps", and "time steps" with respect to a vectorized representation sequence may be used interchangeably.

In an example, each predicted translocation value may indicate how many polymer units the polymer has moved relative to an observation site of the nanopore sequencing at a corresponding time step.

Exemplarily, assuming that the weight vector is [0.5, 0.3, 0.8, 1.3, 0.2], the first element 0.5 may indicate that the polymer has moved 0.5 polymer units relative to a non-specific observation site of the nanopore sequencing at a first time step; the second element 0.3 may indicate that the same polymer has moved 0.3 polymer units relative to the observation site of the nanopore sequencing at a second time step; and so on. It can be understood that, based on the number of elements in the weight vector, the number of steps of the raw signal derived from the polymer during nanopore sequencing is 5. Therefore, the weight vector indicates, at most, how many polymer units the polymer has moved relative to the observation site of the nanopore sequencing up to the fifth time step. That is, in this example, the polymer has moved 0.2 polymer units relative to the observation site at the fifth time step.

Exemplarily, in the case of bases or base fragments, the training and performance evaluation of the first machine learning model may be similar to base or base-fragment translocation prediction algorithms known in the art. Therefore, details of the first machine learning model are not repeated herein so as to avoid obscuring the inventive concept of the present disclosure.

In step S202 of the embodiments, the first vectorized representation sequence may be integrated with the weight vector to obtain a second vectorized representation sequence characterizing the polymer.

It is assumed that the weight vector is [0.5, 0.3, 0.8, 1.3, 0.2]. Exemplarily, a sampling method may be employed to extract elements from the weight vector that are greater than or equal to a threshold. For example, the threshold may be set to 0.5, such that the first, third, and fourth elements in the weight vector are extracted. Subsequently, column vectors $X_1$, $X_3$, and $X_4$ at the corresponding first, third, and fourth time steps may be extracted from the first vectorized representation sequence (it is assumed here that the first vectorized representation sequence is arranged in an N × T format, where T denotes the number of sequence elements or the number of time steps, and N denotes the number of channels) and combined to serve as the second vectorized representation sequence, that is, B = [$X_1$, $X_3$, $X_4$]. In this case, the length (number of steps) of B is L = 3.

Exemplarily, the threshold may be further relaxed to 0.3, thereby extracting the first element to the fourth element in the weight vector. Subsequently, column vectors $X_1$ to $X_4$ at the corresponding first time step to fourth time step may be extracted from the first vectorized representation sequence (it is assumed here that the first vectorized representation sequence is arranged in a T × N format, where T denotes the number of sequence elements or the number of time steps, and N denotes the number of channels) and combined to serve as the second vectorized representation sequence, that is, B = [$X_1$, $X_2$, $X_3$, $X_4$]. In this case, the length (number of steps) of B is L = 4, which is greater by 1 than L in the previous exemplary method.

Exemplarily, weights in the weight vector that are lower than the threshold may be set to 0, or vectorized representations in the first vectorized representation sequence at steps corresponding to elements in the weight vector that are lower than the threshold may be replaced with high-dimensional feature mappings corresponding to an absence of a base, thereby obtaining the second vectorized representation sequence (in this case, it can be seen that the length of the second vectorized representation sequence is the same as that of the first vectorized representation sequence). Additionally, these replaced high-dimensional feature mappings may be concentrated at the beginning or at the end of the second vectorized representation sequence.

It can be understood that the above method for integrating the first vectorized representation sequence with the weight vector is provided only as an example. Any method capable of obtaining, based on both the first vectorized representation sequence and the weight vector, an integrated vectorized representation sequence of the raw signal of nanopore sequencing is feasible, and the present disclosure does not impose any limitation in this regard.

Therefore, the above method 200 is capable of integrating the output of the base translocation prediction module (e.g., the first machine learning model) with high-dimensional information obtained by encoding the raw signal derived from the polymer during nanopore sequencing to obtain high-dimensional features of the raw signal, thereby facilitating a downstream module in outputting a polymer sequence corresponding to the nanopore signal. By virtue of the utilization of the sequence context information during the processing procedure, improved efficiency and accuracy in processing the nanopore signal are achieved.

In an embodiment, the first vectorized representation sequence may be obtained by processing the raw signal by a second machine learning model. Since high-dimensional information carries more information payload than low-dimensional information, processing the low-dimensional raw signal into a high-dimensional vectorized representation sequence through a machine learning model can facilitate processing by a downstream machine learning model, particularly subsequent prediction of polymer unit translocation values, thereby improving processing efficiency and accuracy of nanopore signals.

In an example, the second machine learning model may be any suitable machine learning model, such as a fully connected network, a convolutional neural network, a recurrent neural network, an attention mechanism, or a combination of one or more networks (e.g., an ensemble model). The present disclosure does not impose any limitation in this regard.

In an example, the second machine learning model may refer to an algorithm or mathematical model capable of converting a raw signal derived from a polymer during nanopore sequencing on the polymer into the first vectorized representation sequence characterizing the polymer. In an example, the second machine learning model may output, in response to receiving the raw signal, a first vectorized representation sequence having the same number of steps as the raw signal.

As used herein, the term "step" particularly refers to a sampling point of a discretized signal or an element of a vectorized representation sequence. For the raw signal derived from the polymer during nanopore sequencing, when referring to a step, it may refer to each sampling point of the raw signal, that is, each signal value varying over time included in the raw signal, and the number of steps may refer to the number of sampling points of the raw signal. For a vectorized representation sequence, when referring to a step, it may refer to each element in the sequence, that is, each vectorized representation included in the sequence, and the number of steps may refer to the number of elements included in the sequence or the number of vectorized representations.

In the present disclosure, the terms "step" and "time step" may be used interchangeably.

In an embodiment, the first vectorized representation sequence may be obtained by processing the raw signal by the second machine learning model and subsequently performing downsampling. Therefore, the computational load can be further reduced, and the robustness of nanopore signal processing can be improved.

In an example, raw signals having t time steps may be processed into high-dimensional information (i.e., the first vectorized representation sequence) having a number of steps equal to t/s, where s denotes a downsampling factor.

In an embodiment, the second machine learning model may be an encoder model.

In an example, the encoder model may be an encoder in a transformer-based encoder-decoder model, or the like. The present disclosure does not impose any limitation in this regard.

In an embodiment, the first machine learning model may include:

a first submodel, configured to process the first vectorized representation sequence to obtain a processed first vectorized representation sequence; and
a second submodel, configured to process the processed first vectorized representation sequence to obtain the weight vector.

In an embodiment, the first submodel is a convolutional neural network layer configured to provide context information for processing by the second submodel. Therefore, through the processing of the convolutional neural network, each vectorized representation in the processed first vectorized representation sequence can incorporate information payloads from other vectorized representations in the sequence, thereby providing rich context information for the generation of the weight vector and improving the reliability of nanopore signal processing.

In an embodiment, the first vectorized representation sequence and the second vectorized representation sequence each have a second dimension in addition to length, and the first vectorized representation sequence and the second vectorized representation sequence have the same number of channels in the second dimension.

In an example, the number of channels may correspond to the number of nanopores in a nanopore sequencing instrument that are capable of performing parallel sequencing simultaneously.

Exemplarily, the number of channels N may be any suitable positive integer, e.g., 16, 32, 64, 128, 256, or 512. The present disclosure does not impose any limitation in this regard, and the number of channels is not limited to an integer power of 2.

In an embodiment, integrating the first vectorized representation sequence with the weight vector includes:

processing the first vectorized representation sequence based on corresponding predicted translocation values in the weight vector, while maintaining the number of channels of the first vectorized representation sequence in the second dimension unchanged.

In an embodiment, processing the first vectorized representation sequence based on the corresponding predicted translocation values in the weight vector may include:

for each element in the weight vector, adding the element to all elements preceding the element in position, and using a resulting value as a replacement element for the element to obtain a forward cumulative vector;

in the forward cumulative vector, using elements whose values reach each integer increment of 1 as merge points to obtain a merge vector;

for each merge point in the merge vector, splitting the element at the corresponding position in the weight vector into a portion before the merge point and a portion after the merge point to obtain a decomposed weight vector;

determining merge intervals of the decomposed weight vector based on grouping of elements in the decomposed weight vector; and

weighting vectorized representations at corresponding time steps in the first vectorized representation sequence based on weights within respective merge intervals of the decomposed weight vector to obtain the second vectorized representation sequence.

The embodiment will be described in detail below.

Exemplarily, it is assumed that the weight vector is [0.5, 0.3, 0.8, 1.3, 0.2]. In the first step, forward accumulation is performed. Each element in the weight vector is added to all elements preceding the element in position (if any), and the resulting value is used as a replacement element for the element. Specifically, for the first element 0.5 of the weight vector, since the element is the first among all elements and other elements preceding the element in position do not exist, the first element of the vector obtained after forward accumulation remains 0.5; the second element 0.3 of the weight vector is added to the first element 0.5 preceding the second element in position to obtain a value of 0.8, which is used as the second element of the vector obtained after forward accumulation; the third element 0.8 of the weight vector is added to the first element 0.5 and the second element 0.3 preceding the third element in position to obtain a value of 1.6, which is used as the third element of the vector obtained after forward accumulation; the fourth element 1.3 of the weight vector is added to the first element 0.5, the second element 0.3, and the third element 0.8 preceding the fourth element in position to obtain a value of 2.9, which is used as the fourth element of the vector obtained after forward accumulation; and the fifth element 0.2 of the weight vector is added to the first element 0.5, the second element 0.3, the third element 0.8, and the fourth element 1.3 preceding the fifth element in position to obtain a value of 3.1, which is used as the fifth element of the vector obtained after forward accumulation. Therefore, a forward cumulative vector [0.5, 0.8, 1.6, 2.9, 3.1] is obtained.

In the second step, merge points are determined. In the forward cumulative vector, elements whose values reach each integer increment of 1 are used as merge points to obtain a merge vector. Specifically, the first and second elements of the forward cumulative vector are both less than 1 and therefore are not merge points; the third element 1.6 reaches 1, and can be used as a merge point and replaced with a value of 1; the fourth element 2.9 reaches 2, and can be used as a merge point and replaced with a value of 2; and the fifth element 3.1 reaches 3 and can be used as a merge point and replaced with a value of 3. Therefore, a merge vector [0, 0, 1, 2, 3] is obtained.

In the third step, the weight vector is split based on the merge vector. It is recognized that the merge vector is in a one-to-one correspondence with the elements of the weight vector. For each merge point in the merge vector, the element at the corresponding position in the weight vector is required to be split into a portion before the merge point and a portion after the merge point to obtain a decomposed weight vector. Specifically, for the first and second elements of the weight vector, since the elements at the corresponding positions in the merge vector are not merge points, the first and second elements in the decomposed weight vector remain the original first and second elements 0.5 and 0.3 of the weight vector. For the third element of the weight vector, since the element at the corresponding position in the merge vector is merge point 1, the merge point 1 is first split into two values. The first value is determined such that, when added to all elements preceding the element that is in the weight vector and corresponds to the position of the merge point (i.e., the first and second elements 0.5 and 0.3), the sum equals 1, and the second value is the remainder obtained by subtracting the above first value from the element that is in the weight vector and corresponds to the position of the merge point. In this example, the first value is (1 - 0.5 - 0.3) = 0.2, and the second value is (0.8 - 0.2) = 0.6. For the fourth element of the weight vector, since the element at the corresponding position in the merge vector is merge point 2, the merge point 2 is first split into two values. The first value is determined such that, when added to all elements preceding the element that is in the weight vector and corresponds to the position of the merge point (i.e., the first to third elements 0.5, 0.3, and 0.8), the sum equals 2, and the second value is the remainder obtained by subtracting the above first value from the element that is in the weight vector and corresponds to the position of the merge point. In this example, the first value is (2 - 0.5 - 0.3 - 0.8) = 0.4, and the second value is (1.3 - 0.4) = 0.9. For the fifth element of the weight vector, since the element at the corresponding position in the merge vector is merge point 3, the merge point 3 is first split into two values. The first value is determined such that, when added to all elements preceding the element that is in the weight vector and corresponds to the position of the merge point (i.e., the first to fourth elements 0.5, 0.3, 0.8, and 1.3), the sum equals 3, and the second value is the remainder obtained by subtracting the above first value from the element that is in the weight vector and corresponds to the position of the merge point. In this example, the first value is (3 - 0.5 - 0.3 - 0.8 - 1.3) = 0.1, and the second value is (0.2 - 0.1) = 0.1. Therefore, a split weight vector (i.e., a decomposed weight vector) [0.5, 0.3, 0.2 | 0.6, 0.4 | 0.9, 0.1 | 0.1] can be obtained.

In the fourth step, merge intervals of the split weight vector (i.e., the decomposed weight vector) are determined. The merge intervals represent groupings of elements in the split weight vector, where each merge interval is defined by using corresponding split values of adjacent merge points in the split weight vector as boundaries. For example, the first merge point in the split weight vector is the third element, i.e., 0.2 | 0.6. Although there is no other merge point preceding this merge point, the first element 0.5 may serve as a lower bound of the first merge interval, and the split value 0.2 in the merge point 0.2 | 0.6 may serve as an upper bound of the first merge interval. Therefore, the weights within the first merge interval are obtained, that is, [0.5, 0.3, 0.2]. Similarly, the second merge point in the split weight vector is the fourth element, i.e., 0.4 | 0.9. Before this merge point, the remaining split value 0.6 of the first merge point is present. The split value 0.6 may serve as a lower bound of the second merge interval, and the split value 0.4 in the merge point 0.4 | 0.9 may serve as an upper bound. Therefore, the weights within the second merge interval are obtained, that is, [0.6, 0.4]. Then, the third merge point in the split weight vector is the fifth element, i.e., 0.1 | 0.1. Before this merge point, the remaining split value 0.9 of the second merge point is present. The split value 0.9 may serve as a lower bound of the second merge interval, and the split value 0.1 (the left-side 0.1) in the merge point 0.1 | 0.1 may serve as an upper bound. Therefore, the weights within the third merge interval are obtained, that is, [0.9, 0.1]. It is noted that the sum of all weight values within each merge interval is exactly 1. It is further noted that the other split value 0.1 (the right-side 0.1) of the final merge point 0.1 | 0.1 remains. Since no further elements or merge points and their split values exist in the split weight vector, in this example, the remaining split value 0.1 may be discarded. Therefore, three merge intervals are obtained, that is, [0.5, 0.3, 0.2], [0.6, 0.4], and [0.9, 0.1].

In the fifth step, the vectorized representations at corresponding time steps in the first vectorized representation sequence are weighted based on the weights within the merge intervals of the split weight vector (i.e., the decomposed weight vector) to obtain the second vectorized representation sequence having a length of L.

For ease of understanding, the three merge intervals obtained in the fourth step may be aligned with the steps of the first vectorized representation sequence. Before that, as described above, since the number of predicted translocation values included in the weight vector is the same as the number of elements or the number of steps of the first vectorized representation sequence, it can be determined that the number of steps T of the first vectorized representation sequence is also 5. Assuming that each individual vectorized representation is a column vector of dimension $N \times 1$, the first vectorized representation sequence in this example has a dimension of $N \times 5$. Accordingly, the first vectorized representation sequence A may be denoted as $[X_1, X_2, X_3, X_4, X_5]$, where each column vector $X_n$ ($n \in 1, ..., 5$) may have N elements (i.e., N rows or N channels, N being a positive integer greater than or equal to 1, as described above). Therefore, the aligned merge intervals $C_1$-$C_3$ can be obtained as follows:

$C_1$ = [0.5, 0.3, 0.2, null, null];
$C_2$ = [null, null, 0.6, 0.4, null]; and
$C_3$ = [null, null, null, 0.9, 0.1].

Here, "null" indicates empty, meaning that the element at the corresponding position in the first vectorized representation sequence (i.e., the corresponding vectorized representation) does not need to participate in the weighting operation.

Subsequently, weighting may be performed on the vectorized representations at corresponding time steps of the first vectorized representation sequence based on the respective merge intervals $C_1$-$C_3$. Specifically, $X_1$, $X_2$, and $X_3$ may be multiplied by the non-null elements in $C_1$, respectively, such that the first element in the second vectorized representation sequence having a length of L is obtained, i.e., $0.5X_1 + 0.3X_2 + 0.2X_3$. Similarly, $X_3$ and $X_4$ may be multiplied by the non-null elements in $C_2$, respectively, such that the second element in the second vectorized representation sequence having a length of L is obtained, i.e., $0.6X_3 + 0.4X_4$. $X_4$ and $X_5$ may be multiplied by the non-null elements in $C_3$, respectively, such that the third element in the second vectorized representation sequence having a length of L is obtained, i.e., $0.9X_4 + 0.1X_5$.

Accordingly, a second vectorized representation sequence B having a length (steps) of L = 3 is obtained, that is, B = [$0.5X_1 + 0.3X_2 + 0.2X_3$, $0.6X_3 + 0.4X_4$, $0.9X_4 + 0.1X_5$]. Compared with the length of the first vectorized representation sequence, the length of the obtained second vectorized representation sequence is reduced.

It can be understood that each element in the second vectorized representation sequence B includes context information of several vectorized representations adjacent to each other in the first vectorized representation sequence, which is conducive to improving processing efficiency and accuracy during sequencing of polymer units based on nanopore raw signals.

In addition, the manner of processing the first vectorized representation sequence based on corresponding predicted translocation values in the weight vector is not limited to the foregoing method, and may also include, for example, the threshold-based method described above. The present disclosure does not impose any limitation in this regard.

It should be noted that the length of the second vectorized representation sequence obtained by integrating the weight vector and the first vectorized representation sequence may be equal to or greater than the length of the first vectorized representation sequence. For cases in which the two lengths are equal, reference may be made, for example, to the description of step S202 in the foregoing embodiment. For cases in which the length of the second vectorized representation sequence is increased compared with that of the first vectorized representation sequence, reference may be made to the following example.

Exemplarily, it is assumed that the weight vector is [1.3, 1.4, 0.7, 1.2, 1.9, 1.8]. It can be understood that, in this case, the lengths of the raw signal and the corresponding first vectorized representation sequence are both 6. In the first step, forward accumulation is performed. Each element in the weight vector is added to all elements preceding the element in position (if any), and the resulting value is used as a replacement element for the element. Therefore, a forward cumulative vector [1.3, 2.7, 3.4, 4.6, 6.5, 8.3] is obtained.

In the second step, merge points are determined. In the forward cumulative vector, elements whose values reach each integer increment of 1 are used as merge points to obtain a merge vector [1, 2, 3, 4, 6, 8].

In the third step, the weight vector is split based on the merge vector, such that a split weight vector (i.e., a decomposed weight vector) [1.0 | 0.3, 0.7 | 0.7, 0.3 | 0.4, 0.6 | 0.6, 0.4 | 1.0 | 0.5, 0.5 | 1.0]. can be obtained (one remaining split value 0.3 is discarded because it is less than 1 and no additional split value exists with which it can be combined to form 1).

In the fourth step, merge intervals of the split weight vector (i.e., the decomposed weight vector) are determined, such that 8 merge intervals are obtained: [1.0], [0.3, 0.7], [0.7, 0.3], [0.4, 0.6], [0.6, 0.4], [1.0], [0.5, 0.5], and [1.0].

In the fifth step, the vectorized representations at corresponding time steps in the first vectorized representation sequence are weighted based on the weights within the merge intervals of the split weight vector (i.e., the decomposed weight vector) to obtain the second vectorized representation sequence having a length of L = 8. It can be seen that the length of the obtained second vectorized representation sequence is increased compared with the length of the first vectorized representation sequence.

Exemplarily, the second vectorized representation of the raw signal of nanopore sequencing may be obtained through threshold-based extraction. In an embodiment, processing the first vectorized representation sequence based on the corresponding predicted translocation values in the weight vector may include: determining one or more elements in the weight vector that satisfy a threshold; selecting, from the first vectorized representation sequence, one or more vectorized representations at positions corresponding to the one or more elements in the weight vector that satisfy the threshold; and obtaining the second vectorized representation sequence based on the selected one or more vectorized representations.

In an example, obtaining the second vectorized representation sequence based on the selected one or more vectorized representations may include directly concatenating the selected one or more vectorized representations to obtain the second vectorized representation sequence. Additionally, vectorized representations at positions in the first vectorized representation sequence corresponding to elements in the weight vector that do not satisfy the threshold (for example, are lower than the threshold) may be replaced with default vectorized representations (for example, such default vectorized representations may correspond to meaningless tokens (for example, in the case of a base sequence, a meaningless token does not represent any base or base fragment)). The replaced default vectorized representations may be placed at any position in the second vectorized representation sequence (for example, at the beginning or at the end of the sequence).

Exemplarily, the second vectorized representation of the raw signal of nanopore sequencing may be obtained through attention computation or matrix transformation.

In an embodiment, integrating the first vectorized representation sequence with the weight vector may include obtaining, according to an attention mechanism, a high-dimensional feature matrix corresponding to the first vectorized representation sequence and a feature fusion matrix corresponding to the weight vector; and obtaining the second vectorized representation sequence based on matrix operations performed on the high-dimensional feature matrix and the feature fusion matrix.

In an example, the dimension of the high-dimensional feature matrix A is defined as $T \times C_{in}$, where T denotes the step, and $C_{in}$ denotes the number of channels. After computation by a fully connected network in an attention layer, $AW_q + b_q = V$ and $AW_k + b_k = K$ are obtained, where $W_q$, $W_k$, $b_q$, and $b_k$ denote parameters associated with the query and the key, respectively, and the dimensions of V and K are both $T \times C$. The high-dimensional feature matrix A may be a matrix representation corresponding to the first vectorized representation sequence described above.

The dimension of the fusion feature matrix B is denoted as $L \times 1$ or $L \times C_{in2}$. The matrix B may be a high-dimensional feature after weighted summation, or may be a vector directly integrating predicted translocation values (i.e., the weight vector described above). After computation by the fully connected network in the attention layer, $BW_q + b_q = Q$ is obtained, where the dimension of Q is $L \times C$. C may be the same as or different from $C_{in}$; however, one of the dimensions C of Q, K, and V is identical. The fusion feature matrix B may be a matrix representation corresponding to the weight vector described above.

After attention computation on Q, K, and V, the second vectorized representation sequence can be obtained.

For example, the attention computation may be expressed as Attention

$$(Q, \; K, \; V) = \mathrm{softmax}(\frac{QK^T}{\sqrt{d_k}})V$$ , where the

operators softmax and $\sqrt{d_k}$ do not change the shape (dimension) of the matrix. The above attention computation may also be expressed in the following matrix operation form: $(Q_{[L \times C]}K^T_{[C \times T]})V_{[T \times C]} = [L \times T] \times [T \times C] = [L \times C]$,

that is, the obtained second vectorized representation sequence has a length of L and a channel number of C. The process of attention computation is well known in the art and will not be described herein again.

In an embodiment, the polymer is a base sequence, and the polymer unit is a base or a base combination.

It can be understood that the polymer of the present disclosure is not limited to a base sequence. For example, when the polymer is a polypeptide, the polymer unit may be various types of naturally occurring or artificially synthesized amino acids. As another example, when the polymer is a saccharide, the polymer unit may be various types of monosaccharides, polysaccharides or the like. In addition, the polymer of the present disclosure may also be an inorganic polymer, which will be readily appreciated by those skilled in the art, and the present disclosure does not impose any limitation in this regard.

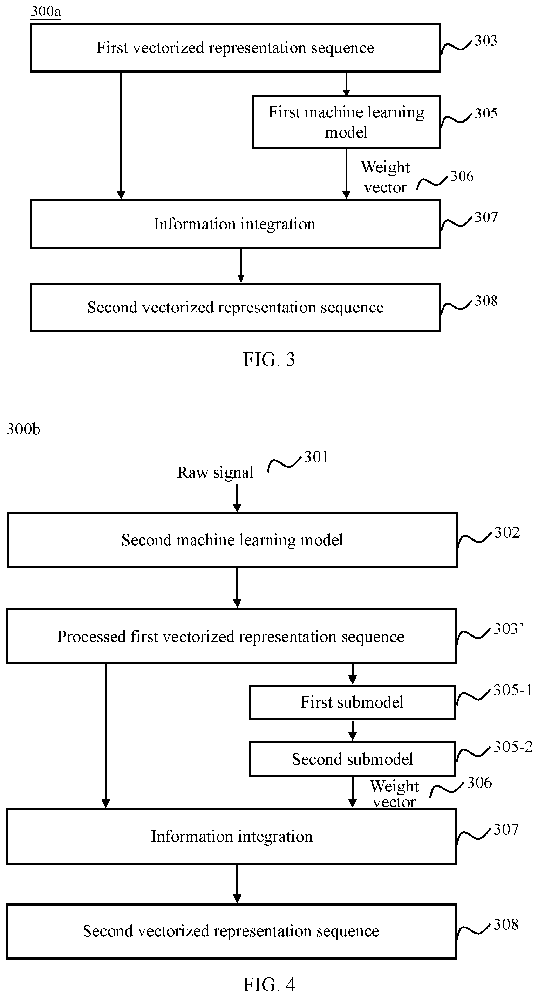

FIGS. 3 and 4 illustrate block diagrams of exemplary processes for processing a first vectorized representation sequence characterizing a polymer according to an exemplary embodiment of the present disclosure. As shown in FIG. 3, an exemplary process 300a begins with a first vectorized representation sequence 303. The first vectorized representation sequence 303 is obtained and input into a first machine learning model 305 to obtain a weight vector 306 output by the first machine learning model 305. The first vectorized representation sequence 303 and the weight vector 306 are subjected to information integration 307 to obtain a second vectorized representation.

The exemplary process 300a may be understood with reference to the description of the method 200 described above, which will not be described herein again.

Referring further to FIG. 4, the exemplary process 300b shown in FIG. 4 differs from the exemplary process 300a in that the processed first vectorized representation sequence 303' is obtained from a raw signal 301 derived from the polymer during nanopore sequencing. Specifically, the raw signal 301 is input into a second machine learning model 302 to obtain the processed first vectorized representation sequence 303' output by the second machine learning model 302. As described above, the second machine learning model 302 may, for example, process the low-dimensional raw signal 301 into a high-dimensional vectorized representation sequence, thus facilitating processing by a downstream machine learning model, particularly subsequent prediction of polymer unit translocation values (for example, the first submodel 305-1 and the second submodel 305-2 shown in FIG. 4), thereby improving processing efficiency and accuracy of nanopore signals. Additionally, the second machine learning model 302 may include a certain degree of downsampling, such that the computational load is reduced, and the robustness of nanopore signal processing is improved. As shown in FIG. 4, the first machine learning model may be further refined into a first submodel 305-1 and a second submodel 305-2. As described above, the first submodel may be configured to further process the processed first vectorized representation sequence (for example, reorganizing the high-dimensional information input into the submodel 305-1 (i.e., the processed first vectorized representation sequence 303'), so as to provide context information for the base translocation prediction process; the first submodel 305-1 may, for example, be a convolutional network layer), to obtain a reprocessed first vectorized representation sequence, and the second submodel may be configured to process the reprocessed first vectorized representation sequence to obtain the weight vector 306.

The exemplary process 300b may be understood with reference to the description of the above method embodiments, which will not be described herein again.

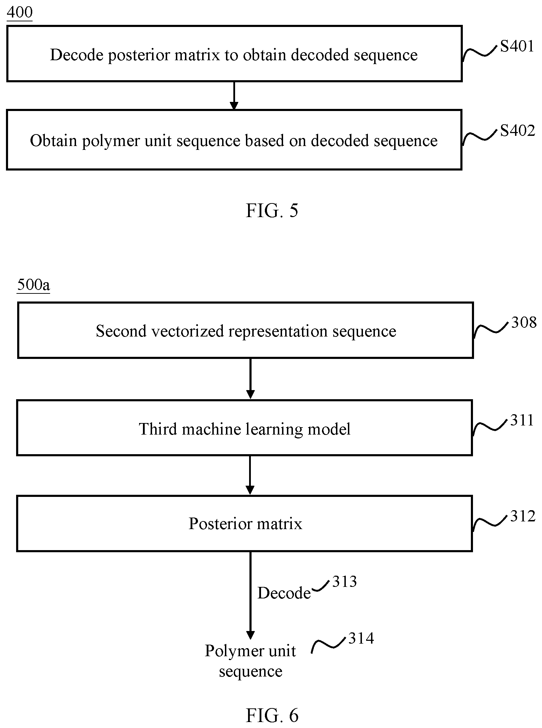

According to another aspect of the present disclosure, a processing method 400 for processing a posterior matrix is provided. As shown in FIG. 5, the processing method 400 includes: step S401, decoding the posterior matrix to obtain a decoded sequence; and step S402, obtaining a polymer unit sequence based on the decoded sequence.

In an example, the posterior matrix may be obtained by processing, by the third machine learning model, the second vectorized representation sequence obtained according to any one of the above method embodiments. The number of rows of the posterior matrix obtained by processing through the third machine learning model is equal to the number of polymer unit classifications, elements in each column of the posterior matrix indicate one corresponding polymer unit among a plurality of polymer units, and elements in a plurality of consecutive columns of the posterior matrix indicate corresponding different polymer units in the plurality of polymer units.

It can be understood that different polymer units refer to individually existing polymer units, rather than indicating that the identifiers of the polymer units are different. For example, according to an embodiment, consecutive m columns of elements of the posterior matrix may indicate m independent (i.e., individually existing) polymer units. Exemplarily, consecutive m columns of elements $P_1$, $P_2$, ..., $P_m$ of the posterior matrix may indicate (e.g., correspond to) polymer units $D_1$, $D_2$, ..., $D_m$, where in the polymer units $D_1$ to $D_m$, there may be a case where identifiers of two or more adjacent polymer units are the same.

In an example, the third machine learning model may be any suitable machine learning model, such as a fully connected network, a convolutional neural network, a recurrent neural network, an attention mechanism, or a combination of one or more networks (e.g., an ensemble model). The present disclosure does not impose any limitation in this regard.

In an example, the third machine learning model may refer to an algorithm or mathematical model capable of converting the second vectorized representation sequence into the posterior matrix.

In an example, the third machine learning model may output, in response to receiving the second vectorized representation sequence, a posterior matrix having a number of columns equal to the steps of the second vectorized representation sequence and a number of rows equal to the number of token classifications.

As used in the present disclosure, the term "token" refers to a basic unit of text. In the context of nanopore sequencing of a polymer, the term particularly refers to a minimum constituent element of a polymer unit sequence. The token may be various polymer unit identifiers used to represent polymer unit types (in the case of a base sequence, for example, adenine is represented by A, guanine is represented by G, cytosine is represented by C, thymine is represented by T, and uracil is represented by U), and may also be various optional markers (for example, a placeholder N, a sequence start symbol, or a sequence end symbol). Accordingly, it can be understood that, in the context of the present disclosure, when referring to various polymer unit sequences, the term may refer either to a polymer unit sequence homogeneous with a polymer unit sequence existing in nature or not yet discovered (i.e., the minimum constituent elements forming the polymer unit sequence only include polymer unit identifiers), or to a polymer unit sequence heterogeneous with a polymer unit sequence existing in nature or not yet discovered (i.e., the minimum constituent elements forming the polymer unit sequence include or only include various markers). In the context of nanopore sequencing of a polymer, tokens are only selected from various polymer unit identifiers and various markers. Accordingly, each polymer unit identifier and each marker constitutes a candidate token, and various polymer unit identifiers and various markers together form a candidate token library.

As used in the present disclosure, the term "posterior matrix" may refer to matrix-form information obtained after taking prior knowledge into consideration and performing operations based on such prior knowledge. In the context of nanopore sequencing of a polymer, the term particularly refers to matrix-form information obtained by performing (various) operations dependent on prior knowledge on a raw signal of nanopore sequencing (for example, processing the first vectorized representation sequence by using a pre-trained first machine learning model, and processing the second vectorized representation sequence by using a pre-trained third machine learning model), and polymer unit determination in a nanopore sequencing scenario can be made based on such matrix-form information.

In step S401 of the embodiment, a decoding operation may be performed on the posterior matrix to obtain a decoded sequence.

Exemplarily, a decoding operation may be separately performed for elements in each column of the posterior matrix.

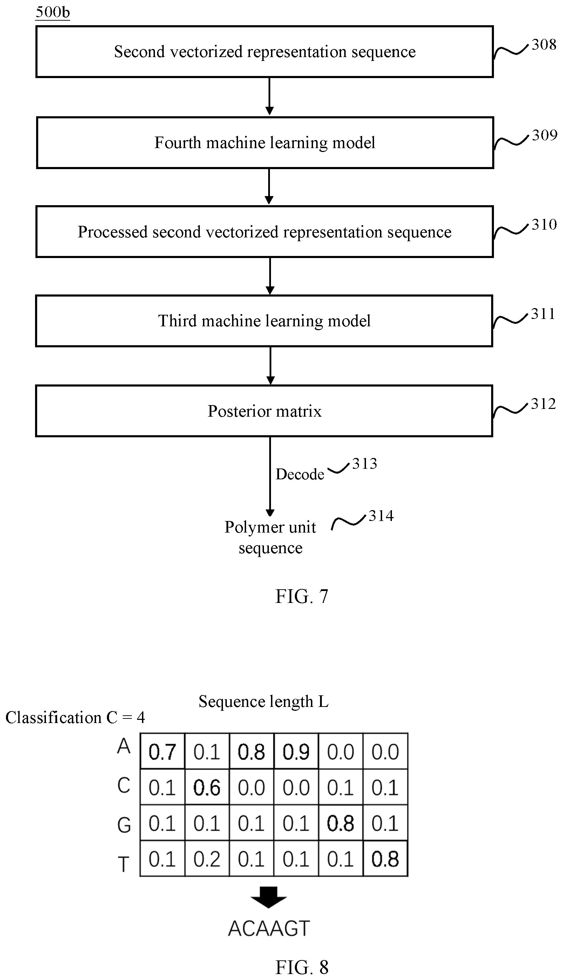

Referring to FIG. 8, FIG. 8 illustrates a diagram of an exemplary process for decoding a posterior matrix according to an exemplary embodiment of the present disclosure.

As shown in FIG. 8, the posterior matrix has a dimension of C × L, where C denotes the number of token categories, and L denotes a sequence length equal to the number of steps of the second vectorized representation sequence. In the illustrated scenario, based on prior knowledge, the token types are bases A, C, G, and T, respectively. That is, the third machine learning model processes the second vectorized representation sequence, based on prior knowledge, into a posterior matrix containing only the above four types of bases. As an example and not by way of limitation, a decoding operation may be separately performed for elements in each column of the illustrated posterior matrix. It can be understood that, when the polymer unit categories included in the posterior matrix are various individual bases, the decoding results of Viterbi decoding, beam search with a beam width of 1, and greedy search (i.e., selecting the polymer category corresponding to the maximum value in elements in each column as the polymer identifier at the step corresponding to that element column) are the same.

Specifically, as shown in FIG. 8, the base type A corresponding to the maximum value 0.7 in the elements in the first column of the posterior matrix is selected as the base ID at step 1 = 1 corresponding to the elements in the first column; the base type C corresponding to the maximum value 0.6 in the elements in the second column of the posterior matrix is selected as the base ID at step 1 = 2 corresponding to the elements in the second column; the base type A corresponding to the maximum value 0.8 in the elements in the third column of the posterior matrix is selected as the base ID at step 1 = 3 corresponding to the elements in the third column; the base type A corresponding to the maximum value 0.9 in the elements in the fourth column of the posterior matrix is selected as the base ID at step 1 = 4 corresponding to the elements in the fourth column; the base type G corresponding to the maximum value 0.8 in the elements in the fifth column of the posterior matrix is selected as the base ID at step 1 = 5 corresponding to the elements in the fifth column; and the base type T corresponding to the maximum value 0.8 in the elements in the sixth column of the posterior matrix is selected as the base ID at step 1 = 6 corresponding to the elements in the sixth column. Therefore, a decoded sequence ACAAGT is obtained.

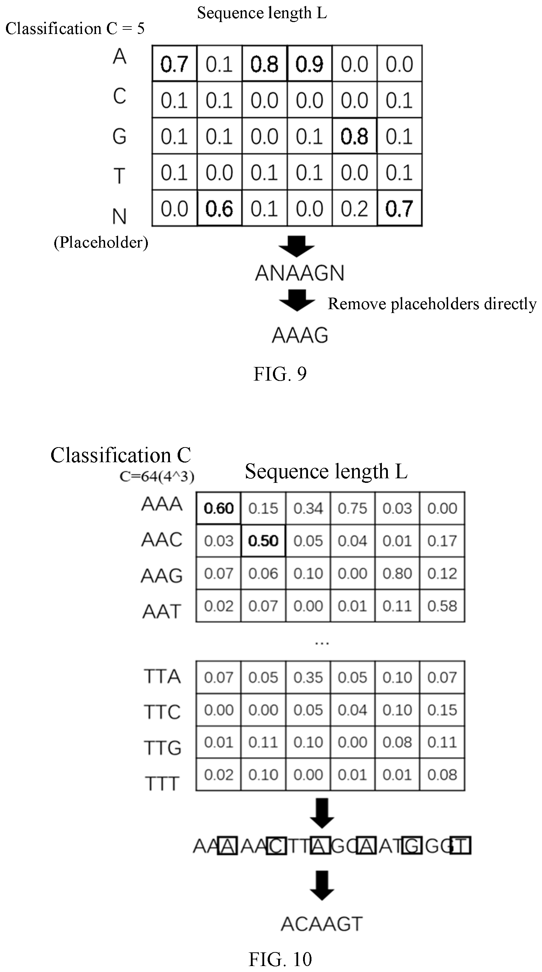

Referring to FIG. 9, FIG. 9 illustrates a diagram of another exemplary process for decoding a posterior matrix according to an exemplary embodiment of the present disclosure.

As shown in FIG. 9, the posterior matrix has a dimension of C × L, where C denotes the number of token categories, and L denotes a sequence length equal to the number of steps of the second vectorized representation sequence. In the illustrated scenario, based on prior knowledge, the token types are bases A, C, G, T, and N, respectively. It can be seen that, compared with the example of FIG. 8, an additional placeholder N is included in the token categories herein. However, this does not affect performing the decoding operation separately for elements in each column of the posterior matrix. It can be understood that, when the polymer unit categories included in the posterior matrix are various individual bases and/or various individual placeholders, the decoding results of Viterbi decoding, beam search with a beam width of 1, and greedy search (i.e., selecting the polymer category corresponding to the maximum value in elements in each column as the polymer identifier at the step corresponding to that element column) are still the same.

Specifically, as shown in FIG. 9, according to the same decoding process as that of FIG. 8, a decoded sequence ANAAGN can be obtained.

In step S402 of the embodiment, a polymer unit sequence can be obtained based on the decoded sequence.

In an example, the decoded sequence may be directly used as the polymer unit sequence. Exemplarily, as shown in FIG. 8, the decoded sequence ACAAGT may be directly used as the polymer unit sequence.

In an example, the decoded sequence may be processed, and a result of the processing may be used as the polymer unit sequence. Exemplarily, as shown in FIG. 9, placeholder tokens in the decoded sequence may be deleted, and the resulting sequence AAAG may be used as the polymer unit sequence.

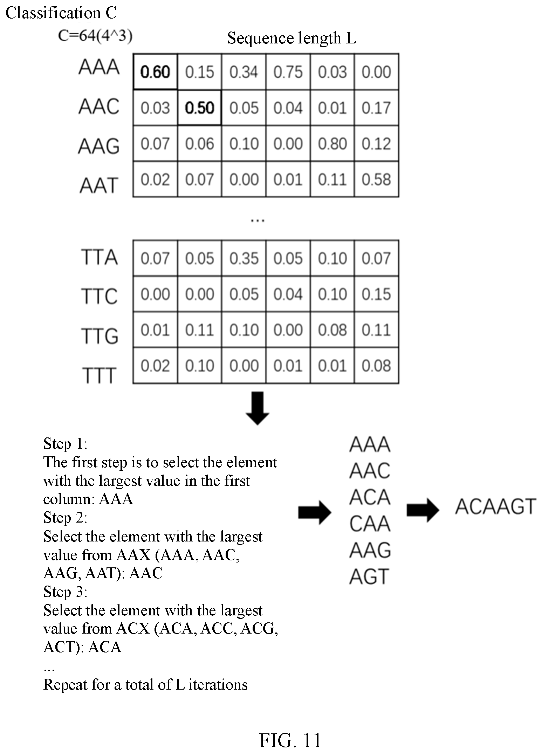

Referring now to FIGS. 10 and 11, FIGS. 10 and 11 illustrate diagrams of another two exemplary processes for decoding a posterior matrix according to an exemplary embodiment of the present disclosure, respectively.

Different from FIGS. 8 and 9, in FIGS. 10 and 11, the polymer categories included in the posterior matrix are no longer individual bases, but base fragments each composed of three bases, and each individual base belongs to one of A, C, G, and T. In other words, there are a total of $4^3 = 64$ base fragment types.

As shown in FIG. 10, similar to the decoding processes of FIGS. 8 and 9, for elements in each column of the posterior matrix, the base fragment type corresponding to the maximum value of the elements is selected as the base fragment identifier at the step corresponding to that element column. For example, the maximum value of the elements in the first column is 0.60, corresponding to the base fragment AAA, and thus AAA is taken as the base fragment ID at the first step; the maximum value of the elements in the second column is 0.50, corresponding to the base fragment AAC, and thus AAC is taken as the base fragment ID at the second step; and so on. Therefore, a decoded sequence AAA AAC TTA GCA ATG GGT is obtained. Assuming that given prior knowledge indicates that the polymer units included in the polymer unit sequence are all base fragments each composed of three bases, the above decoded sequence may be directly used as the polymer unit sequence. Conversely, if given prior knowledge indicates that the polymer units included in the polymer unit sequence include only various individual bases and do not include base fragments, then, for example, the least significant bit position of each base fragment ID decoded from elements in each column of the posterior matrix may be retained, thereby obtaining a final polymer unit sequence. Specifically, as shown in FIG. 10, the rightmost base ID of each base fragment ID in the decoded sequence AAA AAC TTA GCA ATG GGT is retained, thereby obtaining the polymer unit sequence ACAAGT.

In another example, the middle base ID of each base fragment ID in the decoded sequence AAA AAC TTA GCA ATG GGT shown in FIG. 10 may be retained, thereby obtaining a polymer unit sequence AATCTG. In yet another example, the leftmost base ID of each base fragment ID in the decoded sequence AAA AAC TTA GCA ATG GGT shown in FIG. 10 may be retained, thereby obtaining a polymer unit sequence AATGAG. It can be understood that the above various manners of obtaining a final polymer unit sequence based on base fragment IDs decoded from elements in each column of the posterior matrix are exemplary. Those skilled in the art may select, as needed, which base ID in each base fragment ID is to be retained to form the polymer unit sequence, and the present disclosure does not impose any limitation in this regard.

FIG. 11 illustrates an exemplary process of performing Viterbi decoding on a posterior matrix. As shown in FIG. 11, first, for the elements in the first column of the posterior matrix, the base fragment type corresponding to the maximum value 0.60 of the elements is selected as the base fragment identifier AAA at the step corresponding to the elements in the first column. Then, taking overflow into consideration, the base fragment identifier AAA is shifted left by one position to obtain a candidate base fragment pattern AAX at the next step (i.e., the step corresponding to the elements in the second column), where X denotes a base identifier to be determined and may be selected from one of A, C, G, and T. It can be understood that there are four types of base fragments conforming to the base fragment pattern AAX, i.e., AAA, AAC, AAG, and AAT. From elements in the second column of the posterior matrix corresponding to these four types of base fragments, the maximum value 0.50 of the elements is determined, and the base fragment type corresponding to 0.50 is selected as the base fragment identifier AAC at the step corresponding to the elements in the second column. Subsequently, similarly, taking overflow into consideration, the base fragment identifier AAC is shifted left by one position to obtain a candidate base fragment pattern ACX at the next step (i.e., the step corresponding to the elements in the third column), namely ACA, ACC, ACG, and ACT. From elements in the third column of the posterior matrix corresponding to these four types of base fragments, the maximum value of the elements is determined, and the base fragment type corresponding to the maximum value is selected as the base fragment identifier at the step corresponding to the elements in the third column, which in this example is ACA. By analogy, the above procedure is repeated for a total of L iterations to obtain a decoded sequence AAA AAC ACA CAA AAG AGT. Assuming that given prior knowledge indicates that the polymer units included in the polymer unit sequence are all base fragments each composed of three bases, the above decoded sequence may be directly used as the polymer unit sequence. Conversely, if given prior knowledge indicates that the polymer units included in the polymer unit sequence include only various individual bases and do not include base fragments, then, for example, the least significant bit position of each base fragment ID decoded from the L decoding iterations may be retained, thereby obtaining a final polymer unit sequence. Specifically, as shown in FIG. 11, the rightmost base ID of each base fragment ID in the decoded sequence AAA AAC ACA CAA AAG AGT is retained, thereby obtaining the polymer unit sequence ACAAGT.

It should be noted that the accompanying drawings do not illustrate all base fragment classifications for the sake of brevity. As shown in FIGS. 10 and 11, the ellipses indicate that the intermediate 56 base fragment types (i.e., base fragments ACX to TGX) are not illustrated. It can be understood that such omission does not affect the description and understanding of the decoding process.

In an embodiment, decoding the posterior matrix includes one of the following:

decoding the posterior matrix using Viterbi decoding;
decoding the posterior matrix using beam search; and
selecting an extreme value in each column of the posterior matrix to obtain the decoded sequence.

In an embodiment, the polymer unit classifications include placeholders, and obtaining the polymer unit sequence based on the decoded sequence includes removing placeholders in the decoded sequence.

In an embodiment, the polymer units are base combinations, each element in the decoded sequence represents a corresponding base combination, and obtaining the polymer unit sequence based on the decoded sequence includes selecting a base at the same position from each element in the decoded sequence as a corresponding element of the polymer unit sequence. Exemplarily, obtaining the polymer unit sequence based on the decoded sequence may include selecting the rightmost base from each element in the decoded sequence as a corresponding element of the polymer unit sequence, as described above with reference to FIG. 11.

In an embodiment, the third machine learning model includes at least one of the following: a fully connected neural network, a convolutional neural network, a recurrent neural network, or an attention mechanism.

In an embodiment, the method further includes, before decoding the posterior matrix:
inputting the second vectorized representation sequence into a fourth machine learning model to obtain a processed second vectorized representation sequence output by the fourth machine learning model.

Deep learning or machine learning techniques may be introduced to further constrain and integrate information of the second vectorized representation sequence. Exemplarily, the fourth machine learning model may use a conditional random field to constrain transitions of polymer units between adjacent steps, thereby providing more prior knowledge. Exemplarily, certain network layers such as convolutional layers or recurrent neural network layers may also be used to construct the fourth machine learning model. It can be understood that the fourth machine learning model may be any suitable machine learning model, such as a convolutional neural network, a recurrent neural network, an attention mechanism, or a combination of one or more networks (e.g., an ensemble model). The present disclosure does not impose any limitation in this regard.

In an embodiment, the fourth machine learning model is configured to provide prior constraints for the step of decoding the posterior matrix.

In an embodiment, the polymer unit classifications are determined based on one or more of the plurality of polymer units.

Exemplarily, the polymer unit classifications may be determined based on individual polymer units among the plurality of polymer units, such as classifications A, C, G, and T as shown in FIGS. 8 and 9.

Exemplarily, the polymer unit classifications may be determined based on multiple individual polymer units among the plurality of polymer units, such as classifications AAA, AAC, AAG, AAT to TTA, TTC, TTG, and TTT as shown in FIGS. 10 and 11.

FIGS. 6 and 7 illustrate block diagrams of exemplary processes for processing a posterior matrix according to an exemplary embodiment of the present disclosure.

As shown in FIG. 6, an exemplary process 500a begins with a second vectorized representation sequence 308. The second vectorized representation sequence 308 is obtained and input into a third machine learning model 311 to obtain a posterior matrix 312 output by the third machine learning model 311. After a decoding operation 313 is performed on the posterior matrix 312, a polymer unit sequence 314 is finally obtained. The exemplary process 500a may be understood with reference to the description of the processing method 400 described above, which will not be described herein again.

Referring further to FIG. 7, the exemplary process 500b shown in FIG. 7 differs from the exemplary process 500a in that the second vectorized representation sequence 308 further undergoes an additional processing step, namely being input into a fourth machine learning model 309 to obtain a processed second vectorized representation sequence 310 output by the fourth machine learning model 309. Exemplarily, as described above, the fourth machine learning model may be configured to provide prior constraints for the step of decoding the posterior matrix.

The exemplary process 300b may be understood with reference to the description of the above method embodiments, which will not be described herein again.

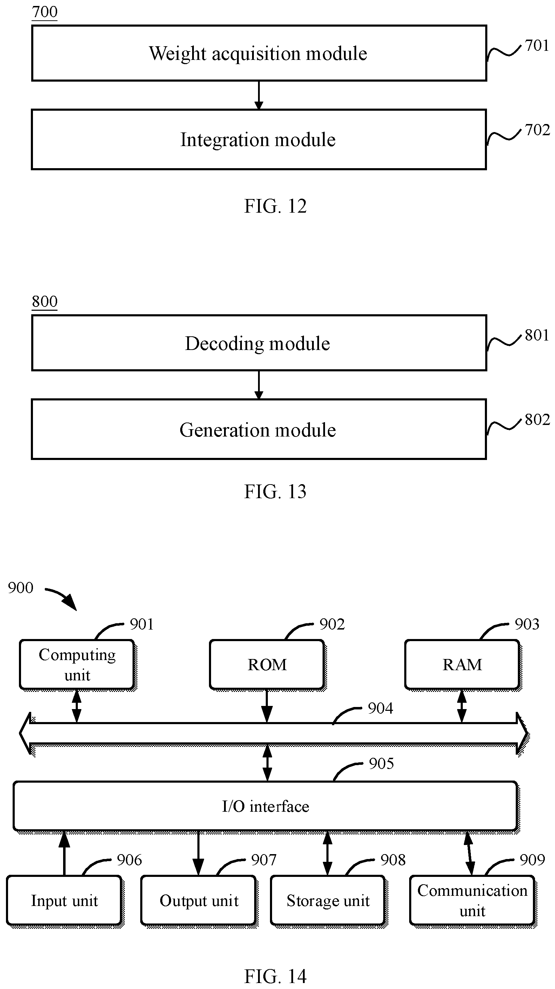

FIG. 12 illustrates a block diagram of an apparatus 700 for processing a first vectorized representation sequence characterizing a polymer according to an exemplary embodiment of the present disclosure.

In an embodiment, the polymer may include a polymer unit sequence, the first vectorized representation sequence may be generated based on a raw signal, and the raw signal may be derived from the polymer by performing nanopore sequencing on the polymer.

As shown in FIG. 12, the apparatus 700 includes a weight acquisition module 701 and an integration module 702.

The weight acquisition module 701 is configured to input the first vectorized representation sequence into a first machine learning model to obtain a weight vector output by the first machine learning model.

In an embodiment, the weight vector may have a same length as the first vectorized representation sequence and may include a plurality of predicted translocation values. Each predicted translocation value may indicate how many polymer units the polymer has moved relative to an observation site of the nanopore sequencing at a corresponding time step.

The integration module 702 is configured to integrate the first vectorized representation sequence with the weight vector to obtain a second vectorized representation sequence characterizing the polymer.

FIG. 13 illustrates a block diagram of an apparatus 800 for processing a posterior matrix according to an exemplary embodiment of the present disclosure.

In an embodiment, the posterior matrix may be obtained by processing, by the third machine learning model, the second vectorized representation sequence obtained according to any one of the above method embodiments. The number of rows of the posterior matrix may be equal to the number of polymer unit classifications. Elements in each column of the posterior matrix may indicate one corresponding polymer unit among a plurality of polymer units, and elements in a plurality of consecutive columns of the posterior matrix may indicate corresponding different polymer units in the plurality of polymer units.

As shown in FIG. 13, the apparatus 800 includes a decoding module 801 and a generation module 802.

The decoding module 801 is configured to decode the posterior matrix to obtain a decoded sequence.

In an embodiment, each element in the decoded sequence corresponds to one polymer unit classification of the polymer unit classifications.

The generation module 802 is configured to obtain a polymer unit sequence based on the decoded sequence.

It should be understood that the respective modules/units of the apparatus 700 and the apparatus 800 shown in FIGS. 12 and 13 may correspond to the respective steps of method 200 and processing method 400 described with reference to FIGS. 2 and 5. Accordingly, the operations, features, and advantages described above with respect to method 200 and processing method 400 are likewise applicable to the apparatus 700 and the apparatus 800 and the modules/units included therein. For brevity, certain operations, features, and advantages will not be described herein again.

While specific functions are discussed above with reference to specific modules, it should be noted that the functions of the various modules discussed herein may be divided into multiple modules, and/or at least some functions of multiple modules may be combined into a single module. Executing an action by a specific module as discussed herein includes the specific module itself executing the action, or alternatively, the specific module invoking or otherwise accessing another component or module that executes the action (or executes the action in conjunction with the specific module). Therefore, the specific module executing an action may include the specific module itself executing the action and/or another module that executes the action and is invoked by or otherwise accessed by the specific module.

It should also be understood that various techniques may be described herein in the general context of software hardware elements or program modules. The various modules and/or units described above with respect to FIGS. 12 and 13 may be implemented in hardware or in hardware combined with software and/or firmware. For example, these modules and/or units may be implemented as computer program codes/instructions, which are configured to be executed in one or more processors and stored in computer-readable storage media. Alternatively, these modules and/or units may be implemented as hardware logic/circuits. One or more of the modules and/or units may be collectively implemented in a system on chip (SoC). The SoC may include integrated circuit chips (including one or a plurality of components of a processor (e.g., a central processing unit (CPU), a microcontroller, a microprocessor, and a digital signal processor (DSP)), a memory, one or a plurality of communication interfaces, and/or other circuits) and may optionally execute the received program code and/or include embedded firmware to perform functions.

According to an embodiment of the present disclosure, a computing device is further provided. The computing device may include at least one processor and a memory in communication connection with the at least one processor. The memory may store one or more instructions executable by the at least one processor, and the one or more instructions may be executed by the at least one processor to enable the at least one processor to perform the method disclosed in any one of the method embodiments described above.

According to an embodiment of the present disclosure, a non-transitory computer-readable storage medium storing one or more computer instructions is further provided. The one or more computer instructions may be used to enable a computer to perform the method disclosed in any one of the method embodiments described above.

According to an embodiment of the present disclosure, a computer program product is further provided. The computer program product may include one or more computer programs. When the one or more computer programs are executed by the processor, the method disclosed in any one of the method embodiments described above can be implemented. According to an embodiment of the present disclosure, an electronic device, a readable storage medium, and a computer program product are further provided. Referring to FIG. 14, a structural block diagram of an electronic device 900 that may serve as the server or the client of the present disclosure will now be described. The electronic device is an example of a hardware device that can be applied to various aspects of the present disclosure. Electronic devices are intended to refer to various forms of digital electronic computer devices, such as laptop computers, desktop computers, workstations, personal digital assistants, servers, blade servers, mainframe computers, and other suitable computers. Electronic devices may also represent various forms of mobile devices, such as personal digital assistants, cellular phones, smartphones, wearable devices, and other similar computing apparatuses. The components shown herein, their connections and relationships, and their functions are examples only and are not intended to limit the implementation of the present disclosure as described and/or claimed herein.

As shown in FIG. 14, the electronic device 900 includes a computing unit 901, which can perform various appropriate actions and processing according to computer programs stored in a read-only memory (ROM) 902 or computer programs loaded from a storage unit 908 into a random-access memory (RAM) 903. In the RAM 903, various programs and data required for the operation of the electronic device 900 can also be stored. The computing unit 901, the ROM 902, and the RAM 903 are connected to each other via a bus 904. An input/output (I/O) interface 905 is also connected to the bus 904.

A plurality of components in the electronic device 900 are connected to the I/O interface 905, including an input unit 906, an output unit 907, the storage unit 908, and a communication unit 909. The input unit 906 may be any type of device capable of inputting information to the electronic device 900. The input unit 906 can receive input digital or character information and generate key signal input related to user settings and/or function control of the electronic device, and may include, but is not limited to, a mouse, a keyboard, a touch screen, a track pad, a trackball, a joystick, a microphone, and/or a remote controller. The output unit 907 may be any type of device capable of presenting information and may include, but is not limited to, a display, a speaker, a video/audio output terminal, a vibrator, and/or a printer. The storage unit 908 may include, but is not limited to, a magnetic disk and an optical disc. The communication unit 909 allows the electronic device 900 to exchange information/data with other devices through a computer network such as the Internet and/or various telecommunication networks, and may include, but is not limited to, a modem, a network card, an infrared communication device, a wireless communication transceiver, and/or a chipset, for example, a bluetooth TM device, an 802.11 device, a WiFi device, a WiMax device, a cellular communication device, and/or the like.

The computing unit 901 may be various general-purpose and/or dedicated processing components having processing and computing capabilities. Some examples of the computing unit 901 include, but are not limited to, a central processing unit (CPU), a graphics processing unit (GPU), various dedicated artificial intelligence (AI) computing chips, various computing units running machine learning network algorithms, a digital signal processor (DSP), and any suitable processor, controller, microcontroller, and the like. The computing unit 901 performs the methods and processes described above, for example, the method for processing a first vectorized representation sequence characterizing a polymer and/or the processing method for processing a posterior matrix, and the embodiments thereof. For example, in some embodiments, a method for training a neural network and/or a method for predicting protein structure may be implemented as a computer software program, which is tangibly embodied in a machine-readable medium, such as the storage unit 908. In some embodiments, part or all of the computer programs may be loaded and/or installed onto the electronic device 900 via the ROM 902 and/or the communication unit 909. When the computer programs are loaded into the RAM 903 and executed by the computing unit 901, one or more steps of the method for processing a first vectorized representation sequence characterizing a polymer and/or the processing method for processing a posterior matrix and the embodiments thereof described above can be performed. Alternatively, in other embodiments, the computing unit 901 may be configured, in any other suitable manners (for example, by means of firmware), to perform the method for processing a first vectorized representation sequence characterizing a polymer and/or the processing method for processing a posterior matrix and the embodiments thereof.

The various implementations of the systems and techniques described above may be implemented in digital electronic circuit systems, integrated circuit systems, field-programmable gate arrays (FPGAs), application-specific integrated circuits (ASIC), application-specific standard products (ASSP), systems on chip (SOCs), complex programmable logic devices (CPLD), computer hardware, firmware, software, and/or combinations thereof. These various implementations may include implementation in one or more computer programs, which can be executed and/or interpreted on a programmable system including at least one programmable processor. The programmable processor may be a dedicated or general-purpose programmable processor that can receive data and instructions from a storage system, at least one input apparatus, and at least one output apparatus, and transmit the data and instructions to the storage system, the at least one input apparatus, and the at least one output apparatus. Program codes for implementing the methods of the present disclosure may be written in any combination of one or more programming languages. These program codes may be provided to a processor or controller of a general-purpose computer, a special-purpose computer, or other programmable data processing apparatuses, such that the program codes, when executed by the processor or controller, enable the implementation of the functions/operations specified in the flowcharts and/or block diagrams. The program codes may be executed entirely on the machine, partly on the machine, as a stand-alone software package, partly on the machine and partly on a remote machine or entirely on the remote machine or server.

In the context of the present disclosure, a machine-readable medium may be a tangible medium that may contain or store programs for use by or in connection with an instruction execution system, an apparatus, or a device. The machine-readable medium may be a machine-readable signal medium or a machine-readable storage medium. The machine-readable medium may include, but is not limited to, an electronic, magnetic, optical, electromagnetic, infrared, or semiconductor system, apparatus, or device, or any suitable combination of the above. More specific examples of the machine-readable storage medium may include one or more wires-based electrical connection, portable computer disk, hard drive, random-access memory (RAM), read-only memory (ROM), erasable programmable read-only memory (EPROM or flash memory), optical fiber, portable compact disk read-only memory (CD-ROM), optical storage device, magnetic storage device, or any suitable combination of the above.

To provide interaction with a user, the systems and techniques described herein may be implemented on a computer equipped with: a display apparatus (e.g., a cathode ray tube (CRT) or a liquid crystal display (LCD) monitor) for displaying information to the user; and a keyboard and a pointing apparatus (e.g., a mouse or a trackball) through which the user can provide input to the computer. Other kinds of apparatuses may also be configured to provide interaction with the user; for example, the feedback provided to the user may be any form of sensory feedback (e.g., visual feedback, auditory feedback, or tactile feedback); and input from the user may be received in any form (including acoustic input, speech input, or tactile input).

The systems and techniques described herein may be implemented in a computing system that includes back-end components (e.g., as a data server), or a computing system that includes middleware components (e.g., an application server), or a computing system that includes front-end components (e.g., a user's computer having a graphical user interface or web browser through which the user can interact with implementations of the systems and techniques described herein), or a computing system that includes any combination of such back-end components, middleware components, or front-end components. The components of the system may be interconnected by any form or medium of digital data communication (e.g., a communication network). Examples of communication networks include: local area network (LAN), wide area network (WAN), and the Internet.

The computer system may include clients and servers. Clients and servers are generally remote from each other and typically interact over a communication network. The relationship between clients and servers is created by computer programs running on corresponding computers and having a client-server relationship with each other. The server may be a cloud server, also known as a cloud computing server or a cloud host. It is a host product in the cloud computing service system to solve the problems of difficult management and weak business scalability in conventional physical hosts and virtual private server services (or VPS for short). The server may also be a server in a distributed system, or a server integrated with a blockchain.

It should be understood that various forms of the process shown above may be used, with steps reordered, added or deleted. For example, the steps disclosed in the present disclosure may be performed in parallel, sequentially, or in a different order, which is not limited herein as long as the desired results of the technical solutions disclosed in the present disclosure can be achieved.

While embodiments or examples of the present disclosure have been described with reference to the accompanying drawings, it should be understood that the methods, systems, and devices described above are merely exemplary embodiments or examples, and that the scope of the present disclosure is not limited by these embodiments or examples, but only limited by the claims as granted and equivalents thereof. Various elements in the embodiments or examples may be omitted or replaced by their equivalent elements. In addition, the steps may be performed in an order different from that described in the present disclosure. Further, the elements in the embodiments or examples may be combined in various ways. It is important to note that as technology evolves, many of the elements described herein may be replaced by equivalent elements that appear after the present disclosure.

## Claims

**1.** A method for processing a first vectorized representation sequence characterizing a polymer, wherein the polymer comprises a polymer unit sequence, the first vectorized representation sequence is generated based on a raw signal, and the raw signal is derived from the polymer by performing nanopore sequencing on the polymer, the method comprising:
inputting the first vectorized representation sequence into a first machine learning model to obtain a weight vector output by the first machine learning model, wherein the weight vector has a same length as the first vectorized representation sequence and comprises a plurality of predicted translocation values, each predicted translocation value indicating how many polymer units the polymer has moved relative to an observation site of the nanopore sequencing at a corresponding time step; and
integrating the first vectorized representation sequence with the weight vector to obtain a second vectorized representation sequence characterizing the polymer.

**2.** The method according to claim 1, wherein the first vectorized representation sequence is obtained by processing the raw signal by a second machine learning model.

**3.** The method according to claim 1, wherein the first vectorized representation sequence is obtained after processing the raw signal by a second machine learning model and subsequently performing downsampling.

**4.** The method according to claim 2 or 3, wherein the second machine learning model is an encoder model.

**5.** The method according to any one of claims 1 to 3, wherein the first machine learning model comprises:

a first submodel, configured to process the first vectorized representation sequence to obtain a processed first vectorized representation sequence; and
a second submodel, configured to process the processed first vectorized representation sequence to obtain the weight vector.

**6.** The method according to claim 5, wherein the first submodel is a convolutional neural network layer configured to provide context information for processing by the second submodel.

**7.** The method according to any one of claims 1 to 3, wherein the first vectorized representation sequence and the second vectorized representation sequence each have a second dimension in addition to length, and the first vectorized representation sequence and the second vectorized representation sequence have a same number of channels in the second dimension.

8.  The method according to claim 7, wherein integrating the first vectorized representation sequence with the weight vector comprises:
processing the first vectorized representation sequence based on corresponding predicted translocation values in the weight vector, while maintaining the number of channels of the first vectorized representation sequence in the second dimension unchanged.

9.  The method according to claim 8, wherein processing the first vectorized representation sequence based on the corresponding predicted translocation values in the weight vector comprises:

for each element in the weight vector, adding the element to all elements preceding the element in position, and using a resulting value as a replacement element for the element to obtain a forward cumulative vector;
in the forward cumulative vector, using elements whose values reach each integer increment of 1 as merge points to obtain a merge vector;
for each merge point in the merge vector, splitting an element at a corresponding position in the weight vector into a portion before the merge point and a portion after the merge point to obtain a decomposed weight vector;
determining merge intervals of the decomposed weight vector based on grouping of elements in the decomposed weight vector; and
weighting vectorized representations at corresponding time steps in the first vectorized representation sequence based on weights within respective merge intervals of the decomposed weight vector to obtain the second vectorized representation sequence.

10. The method according to claim 8, wherein processing the first vectorized representation sequence based on the corresponding predicted translocation values in the weight vector comprises:

determining one or more elements in the weight vector that satisfy a threshold;
selecting, from the first vectorized representation sequence, one or more vectorized representations at positions corresponding to the one or more elements in the weight vector that satisfy the threshold; and
obtaining the second vectorized representation sequence based on the selected one or more vectorized representations.

11. The method according to any one of claims 1 to 3, wherein integrating the first vectorized representation sequence with the weight vector comprises:

obtaining, according to an attention mechanism, a high-dimensional feature matrix corresponding to the first vectorized representation sequence and a feature fusion matrix corresponding to the weight vector; and
obtaining the second vectorized representation sequence based on matrix operations performed on the high-dimensional feature matrix and the feature fusion matrix.

12. The method according to any one of claims 1 to 3, wherein the polymer is a base sequence, and the polymer unit is a base or a base combination.

13. A processing method for processing a posterior matrix, wherein the posterior matrix is obtained by processing, by a third machine learning model, the second vectorized representation sequence obtained by using the method according to any one of claims 1 to 12, a number of rows of the posterior matrix is equal to a number of polymer unit classifications, elements in each column of the posterior matrix indicate one corresponding polymer unit among a plurality of polymer units, and elements in a plurality of consecutive columns of the posterior matrix indicate corresponding different polymer units in the plurality of polymer units, the processing method comprising:

decoding the posterior matrix to obtain a decoded sequence, each element in the decoded sequence corresponding to one polymer unit classification of the polymer unit classifications; and
obtaining the polymer unit sequence based on the decoded sequence.

14. The method according to claim 13, wherein decoding the posterior matrix comprises one of the following:

decoding the posterior matrix using Viterbi decoding;
decoding the posterior matrix using beam search; and
selecting an extreme value in each column of the posterior matrix to obtain the decoded sequence.

15. The method according to claim 13, wherein the polymer unit classifications comprise placeholders, and obtaining the polymer unit sequence based on the decoded sequence comprises removing placeholders in the decoded sequence.

16. The method according to claim 13, wherein the polymer units are base combinations, each element in the decoded sequence represents a corresponding base combination, and obtaining the polymer unit sequence based on the decoded sequence comprises selecting a base at a same position from each element in the decoded sequence as a corresponding element of the polymer unit sequence.

17. The method according to any one of claims 13 to 16, wherein the third machine learning model comprises at least one of the following: a fully connected neural network, a convolutional neural network, a recurrent neural network, or an attention mechanism.

18. The method according to any one of claims 13 to 16, further comprising, before decoding the posterior matrix:
inputting the second vectorized representation sequence into a fourth machine learning model to obtain a processed second vectorized representation sequence output by the fourth machine learning model.

19. The method according to claim 18, wherein the fourth machine learning model is configured to provide prior constraints for the step of decoding the posterior matrix.

20. The method according to any one of claims 13 to 16, wherein the polymer unit classifications are determined based on one or more of the plurality of polymer units.

21. An apparatus for processing a first vectorized representation sequence characterizing a polymer, comprising:

a weight acquisition module, configured to input the first vectorized representation sequence into a first machine learning model to obtain a weight vector output by the first machine learning model, wherein the weight vector has a same length as the first vectorized representation sequence and comprises a plurality of predicted translocation values, each predicted translocation value indicating how many polymer units the polymer has moved relative to an observation site of nanopore sequencing at a corresponding time step; and
an integration module, configured to integrate the first vectorized representation sequence with the weight vector to obtain a second vectorized representation sequence characterizing the polymer.

22. An apparatus for processing a posterior matrix, wherein the posterior matrix is obtained by processing, by a third machine learning model, the second vectorized representation sequence obtained by using the method according to any one of claims 1 to 12, a number of rows of the posterior matrix is equal to a number of polymer unit classifications, elements in each column of the posterior matrix indicate one corresponding polymer unit among a plurality of polymer units, and elements in a plurality of consecutive columns of the posterior matrix indicate corresponding different polymer units in the plurality of polymer units, the apparatus comprising:

a decoding module, configured to decode the posterior matrix to obtain a decoded sequence, each element in the decoded sequence corresponding to one polymer unit classification of the polymer unit classifications; and
a generation module, configured to obtain the polymer unit sequence based on the decoded sequence.

23. A computing device, comprising:

at least one processor; and
at least one memory having one or more code instructions stored thereon, wherein the one or more code instructions, when executed by the at least one processor, cause the computing device to perform the method according to any one of claims 1 to 20.

24. A computer-readable storage medium having one or more code instructions stored thereon, wherein the one or more code instructions, when executed by one or more processors, cause the one or more processors to perform the method according to any one of claims 1 to 20.

25. A computer program product comprising one or more code instructions, wherein the one or more code instructions, when executed by one or more processors, cause the one or more processors to perform the method according to any one of claims 1 to 20.

FIG. 1

200

| Input first vectorized representation sequence into first machine learning model to obtain weight vector output by first machine learning model | S201 |

| Integrate first vectorized representation sequence with weight vector to obtain second vectorized representation sequence characterizing polymer | S202 |

FIG. 2

300a

First vectorized representation sequence ⟋303

First machine learning model ⟋305

Weight vector ⟋306

Information integration ⟋307

Second vectorized representation sequence ⟋308

FIG. 3

300b

Raw signal ⟋301

Second machine learning model ⟋302

Processed first vectorized representation sequence ⟋303'

First submodel ⟋305-1

Second submodel ⟋305-2

Weight vector ⟋306

Information integration ⟋307

Second vectorized representation sequence ⟋308

FIG. 4

400

| Decode posterior matrix to obtain decoded sequence | S401 |

↓

| Obtain polymer unit sequence based on decoded sequence | S402 |

FIG. 5

500a

| Second vectorized representation sequence | 308 |

↓

| Third machine learning model | 311 |

↓

| Posterior matrix | 312 |

↓ Decode 313

Polymer unit 314
sequence

FIG. 6

500b

| Second vectorized representation sequence | ~308 |

↓

| Fourth machine learning model | ~309 |

↓

| Processed second vectorized representation sequence | ~310 |

↓

| Third machine learning model | ~311 |

↓

| Posterior matrix | ~312 |

↓ Decode ~313

Polymer unit sequence ~314

FIG. 7

Sequence length L

Classification C = 4

| | | | | | | |
|---|---|---|---|---|---|---|
| A | 0.7 | 0.1 | 0.8 | 0.9 | 0.0 | 0.0 |
| C | 0.1 | 0.6 | 0.0 | 0.0 | 0.1 | 0.1 |
| G | 0.1 | 0.1 | 0.1 | 0.1 | 0.8 | 0.1 |
| T | 0.1 | 0.2 | 0.1 | 0.1 | 0.1 | 0.8 |

⬇

ACAAGT

FIG. 8

Sequence length L

Classification C = 5

| | | | | | | |
|---|---|---|---|---|---|---|
| A | 0.7 | 0.1 | 0.8 | 0.9 | 0.0 | 0.0 |
| C | 0.1 | 0.1 | 0.0 | 0.0 | 0.0 | 0.1 |
| G | 0.1 | 0.1 | 0.0 | 0.1 | 0.8 | 0.1 |
| T | 0.1 | 0.0 | 0.1 | 0.1 | 0.0 | 0.1 |
| N | 0.0 | 0.6 | 0.1 | 0.0 | 0.2 | 0.7 |

(Placeholder)

ANAAGN

Remove placeholders directly

AAAG

FIG. 9

Classification C

C=64(4^3)

Sequence length L

| | | | | | | |
|---|---|---|---|---|---|---|
| AAA | 0.60 | 0.15 | 0.34 | 0.75 | 0.03 | 0.00 |
| AAC | 0.03 | 0.50 | 0.05 | 0.04 | 0.01 | 0.17 |
| AAG | 0.07 | 0.06 | 0.10 | 0.00 | 0.80 | 0.12 |
| AAT | 0.02 | 0.07 | 0.00 | 0.01 | 0.11 | 0.58 |

...

| | | | | | | |
|---|---|---|---|---|---|---|
| TTA | 0.07 | 0.05 | 0.35 | 0.05 | 0.10 | 0.07 |
| TTC | 0.00 | 0.00 | 0.05 | 0.04 | 0.10 | 0.15 |
| TTG | 0.01 | 0.11 | 0.10 | 0.00 | 0.08 | 0.11 |
| TTT | 0.02 | 0.10 | 0.00 | 0.01 | 0.01 | 0.08 |

AAAAACTTAGCAATGGGT

ACAAGT

FIG. 10

Classification C

C=64(4^3)                          Sequence length L

| | | | | | | |
|---|---|---|---|---|---|---|
| AAA | **0.60** | 0.15 | 0.34 | 0.75 | 0.03 | 0.00 |
| AAC | 0.03 | **0.50** | 0.05 | 0.04 | 0.01 | 0.17 |
| AAG | 0.07 | 0.06 | 0.10 | 0.00 | 0.80 | 0.12 |
| AAT | 0.02 | 0.07 | 0.00 | 0.01 | 0.11 | 0.58 |

...

| | | | | | | |
|---|---|---|---|---|---|---|
| TTA | 0.07 | 0.05 | 0.35 | 0.05 | 0.10 | 0.07 |
| TTC | 0.00 | 0.00 | 0.05 | 0.04 | 0.10 | 0.15 |
| TTG | 0.01 | 0.11 | 0.10 | 0.00 | 0.08 | 0.11 |
| TTT | 0.02 | 0.10 | 0.00 | 0.01 | 0.01 | 0.08 |

Step 1:
The first step is to select the element with the largest value in the first column: AAA
Step 2:
Select the element with the largest value from AAX (AAA, AAC, AAG, AAT): AAC
Step 3:
Select the element with the largest value from ACX (ACA, ACC, ACG, ACT): ACA
...
Repeat for a total of L iterations

AAA
AAC
ACA
CAA
AAG
AGT

ACAAGT

FIG. 11

700

| Weight acquisition module | 701 |

↓

| Integration module | 702 |

FIG. 12

800

| Decoding module | 801 |

↓

| Generation module | 802 |

FIG. 13

900

| Computing unit | 901 |
| ROM | 902 |
| RAM | 903 |

904

| I/O interface | 905 |

| Input unit | 906 | | Output unit | 907 | | Storage unit | 908 | | Communication unit | 909 |

FIG. 14

## INTERNATIONAL SEARCH REPORT

| International application No. |
|---|
| **PCT/CN2023/135133** |

| A. | CLASSIFICATION OF SUBJECT MATTER |
|---|---|

G16B 40/10(2019.01)i

According to International Patent Classification (IPC) or to both national classification and IPC

| B. | FIELDS SEARCHED |
|---|---|

Minimum documentation searched (classification system followed by classification symbols)

IPC:G16B

Documentation searched other than minimum documentation to the extent that such documents are included in the fields searched

Electronic data base consulted during the international search (name of data base and, where practicable, search terms used)

CNTXT; CNABS; VEN; USTXT; EPTXT; WOTXT; CNKI; IEEE: 聚合物, 碱基, 化合物, 氨基酸, 核苷酸, 多糖, 向量, 矩阵, 易位, 位移, 移位, 移动, 预测, 纳米孔, 测序, 序列, 融合, 整合, polymer, nucleobase, compound, amino acid, nucleotide, DNA, polysaccharide, vector, matrix, translocation, displacement, move, predict, nanopore, sequence, fusion, integration

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 116884503 A (BEIJING QITAN TECHNOLOGY CO., LTD. et al.) 13 October 2023 (2023-10-13) claims 1-25, and description, paragraphs 14-176 | 1-25 |
| A | CN 113166804 A (OXFORD NANOPORE TECHNOLOGIES PLC) 23 July 2021 (2021-07-23) description, paragraphs 79-421 | 1-25 |
| A | CN 112183486 A (SUN YAT-SEN UNIVERSITY) 05 January 2021 (2021-01-05) description, paragraphs 64-148 | 1-25 |
| A | US 2005136408 A1 (TOM-MOY, M. et al.) 23 June 2005 (2005-06-23) entire document | 1-25 |

☐ Further documents are listed in the continuation of Box C.  ☑ See patent family annex.

| * | Special categories of cited documents: | | |
|---|---|---|---|
| "A" | document defining the general state of the art which is not considered to be of particular relevance | "T" | later document published after the international filing date or priority date and not in conflict with the application but cited to understand the principle or theory underlying the invention |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **08 May 2024** | **11 May 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| CN | 116884503 | A | 13 October 2023 | CN | 116884503 | B | 26 December 2023 |
| CN | 113166804 | A | 23 July 2021 | JP | 2022509589 | A | 21 January 2022 |
| | | | | AU | 2019389841 | A1 | 20 May 2021 |
| | | | | EP | 3887543 | A1 | 06 October 2021 |
| | | | | WO | 2020109773 | A1 | 04 June 2020 |
| | | | | US | 2020176082 | A1 | 04 June 2020 |
| | | | | SG | 11202103410 | TA | 29 June 2021 |
| | | | | JP | 2023126856 | A | 12 September 2023 |
| | | | | BR | 112021008198 | A2 | 03 August 2021 |
| | | | | CA | 3118632 | A1 | 04 June 2020 |
| | | | | GB | 201819378 | D0 | 09 January 2019 |
| | | | | KR | 20210095641 | A | 02 August 2021 |
| CN | 112183486 | A | 05 January 2021 | CN | 112183486 | B | 01 August 2023 |
| US | 2005136408 | A1 | 23 June 2005 | EP | 1544310 | A2 | 22 June 2005 |
| | | | | EP | 1544310 | A3 | 09 August 2006 |